# EUROPÄISCHE PATENTANMELDUNG

(11) **EP 0 536 645 A2**
(43) Veröffentlichungstag der Anmeldung: **14.04.1993**
(21) Anmeldenummer: 92116841.5
(22) Anmeldetag: 01.10.1992
(51) Int. Cl.: A61M 1/16

(54) **Bereitstellung der bei der Bikarbonat-Hämodialyse benötigten Bikarbonatlösung am Dialysegerät**

(30) Priorität: 11.10.1991 DE 4133652; 04.03.1992 DE 9202829 U; 25.06.1992 DE 9208498 U
(71) Anmelder: Saueressig, Ulrich, Dr. med., D-42103 Wuppertal (DE)
(72) Erfinder: Weiss, Peter, W-5600 Wuppertal 1 (DE); Falkenroth, Frank, W-5657 Haan (DE)
(74) Vertreter: Palgen, Peter, Dr. Dipl.-Phys.

(57) **Zusammenfassung**

Ein Verfahren zur Bereitstellung der bei der Bikarbonat-Hämodialyse benötigten Bikarbonatlösung am Dialysegerät (1O). Eine in einem Behälter (2O) vorhandene berechnete Menge einer festen Bikarbonatzusammensetzung (6) wird mit gereinigtem Wasser zu einer gesättigten Lösung aufgelöst, die von dem Dialysegerät (1O) bedarfsweise aus dem Behälter (2O) entnommen wird. Während der Dialyse erfolgen ständig eine Wiederauffüllung des Behälters (2O) durch gereinigtes Wasser bis zu einer begrenzten Menge und eine fortschreitende Auflösung der Bikarbonatzusammensetzung bis zur Sättigung der Lösung.

## Beschreibung

Die Erfindung bezieht sich auf ein Verfahren der dem Oberbegriff des Anspruchs 1 entsprechenden Art, einen dafür geeigneten Behälter und eine Vorrichtung zur Durchführung des Verfahrens mit dem Behälter.

Bei der Hämodialyse werden relativ große Mengen von Dialysierflüssigkeit benötigt. Je nach Dauer der Behandlung muß mit 150 bis 180 Litern pro Dialyse gerechnet werden. Da es einen erheblichen Transportaufwand bedeutet, derartige Flüssigkeitsmengen in einem vom Dialyseort entfernten pharmazeutischen Betrieb in der Endverdünnung herzustellen und an den Dialyseort zu transportieren, wurde schon frühzeitig dazu übergegangen, Dialysierflüssigkeit in Form von hochkonzentrierten Lösungen bereitzustellen.

Eine Verfahrensweise dabei war, Trockenkonzentrat in größeren Mengen, z.B. in Säcken von 10 oder 25 kg Inhalt, an die Dialysestationen zu liefern, wo in einem besonderen Mischer ein flüssiges Konzentrat hergestellt und über Leitungen an die einzelnen an den Patientenbetten aufgestellten Dialysegeräte gepumpt wurde, wo die Endverdünnung stattfand. Diese Verfahrensweise bedeutet einen erheblichen Einrichtungsaufwand und bringt durch das Vorhandensein des ausgedehnten Leitungssystems Kontaminationsprobleme mit sich.

Eine alternative Verfahrensweise war die Anlieferung von flüssigen Dialysekonzentraten in Kunststoffkanistern, beispielsweise aus Polyäthylen und mit einem Fassungsvermögen von 10 Liter. Diese Kanister werden am Dialysegerät aufgestellt, und es wird das Konzentrat im Dialysegerät auf die Endverdünnung gebracht.

Bei dieser Verfahrensweise stellt die Entsorgung der vielen ziemlich großen Kunststoffkanister das bedeutendste Problem dar.

Eine weitere Entwicklungsstufe stellt die Anlieferung von auf jeweils eine Dialyse berechneten Mengen von Trockenkonzentrat in Kunststoffpatronen dar, die beispielsweise 650 g des betreffenden Salzes fassen. Die Patronen werden von gereinigtem Wasser durchströmt, wobei sich ihr Inhalt auflöst. Das gereinigte Wasser steht an den entsprechenden Dialysegeräten zur Verfügung und wird durch Umkehrosmose von sauberem Leitungswasser in hoher Qualität gewonnen, so daß es weitestgehend von allen Inhaltsstoffen befreit ist.

Der vorbeschriebene Stand der Technik geht aus dem Buch "Grundlagen für Heimdialyse e.V. Wiss. Beratung Dr. Becker u. Prof. Dr. Schoeppe (1989) Kap. 14.4 und aus der EP-A2 278 100 hervor.

Besonders in Betracht kommt der Patronengedanke bei der Bikarbonat-Hämodialyse. Hierbei werden zwei Teilkonzentrate benötigt, von denen eines Ionen Na⁺, K⁺, Ca²⁺ und Mg²⁺ und das andere das Natriumhydrogencarbonat enthält. Wegen der geringen Löslichkeit von Kalziumkarbonat würde bei einer frühzeitigen Zusammenbringung der beiden Komponenten das Kalziumkarbonat ausfallen.

Wenn das das Bikarbonat enthaltende sogenannte basische Trockenkonzentrat in Kartuschen angeliefert wird, bleibt eine solche Kartusche nach jeder Dialyse übrig und bedarf der Entsorgung, da sie nicht wiederverwendungsfähig ist.

Ein weiterer Nachteil des Kartuschenverfahrens ist eine gewisse Unwirtschaftlichkeit, da bei Dialysen, bei denen nicht der gesamte Inhalt aufgebraucht wird, der Restinhalt verworfen werden muß.

Schließlich ist, da die Kartuschen fabrikmäßig hergestellt werden, eine Anpassung der Zusammensetzung der Dialysierflüssigkeit an individuelle Patienten nicht möglich.

Aus der DE-U-81 09 529 ist ein Verfahren bekannt, bei dem in einer zentralen Versorgungseinheit aus trockenen Substanzen nach einer jeweils vorgegebenen Rezeptur chargenweise die für jeweils einen Patienten benötigte Menge hergestellt und in das mit einem entsprechenden Tank ausgerüstete Dialysegerät überführt wird. Hierbei ist zwar eine individuelle Rezeptur möglich, doch ist die zentrale Versorgungseinheit ein zusätzlich erforderliches Aggregat und sind auch bestimmte, mit Tank versehene Dialysegeräte notwendig.

Der Erfindung liegt die Aufgabe zugrunde, die Bereitstellung der Bikarbonatlösung für die BikarbonatHämodialyse einfach und wirtschaftlich zu gestalten.

Diese Aufgabe wird durch die in Anspruch 1 wiedergegebene Erfindung gelöst.

Es wird also der Gedanke der Bereitstellung des Konzentrats in flüssiger Form verlassen und aus einem größerem Vorrat von Trockenkonzentrat gearbeitet, allerdings ohne aufwendige Mischeinrichtung für große Mengen, sondern unter Bereitstellung nur der für eine Dialyse benötigten Einzelmenge, die natürlich dabei auch verlustfrei abgemessen und angepaßt werden kann. Der Behälter ist im Gegensatz zu den zu verwerfenden Kunststoffkartuschen wiederverwendbar und wird immer wieder gereinigt und neu befüllt. Auf diese Weise wird das Problem der Entsorgung der vielen Kartuschen vermieden und auch kostengünstiger gearbeitet, weil die zu verwerfenden Kartuschen einen nicht unbeachtlichen Kostenfaktor darstellen. Dabei bedarf es aber im Gegensatz zum Stand der Technik für die Herstellung der Dialyseflüssigkeit keiner besonderen zentralen Versorgungseinheit und auch keiner großvolumigen Tanks zur Aufnahme der für eine Dialyse benötigten Gesamtmenge an Dialysierflüssigkeit, sondern es wird die in dem Behälter befindliche berechnete Menge an Bikarbonat, die ständig mit einer dem relativ geringen Behältervolumen entsprechenden Wassermenge in Berührung steht, fortschreitend aufgelöst, bis die durch die Wassermenge gebildete Lösung gesättigt ist. Diese gesättigte Lösung wird vom Dialysegerät entnommen und auf die benötigte Konzentration weiterverdünnt, was wegen der konstanten Konzentration der gesättigten Lösung durch Beimischung einer proportionalen Wassermenge einfach, d.h. ohne Konzentrationsmessung möglich ist. Wird die Entnahme unterbrochen, löst sich auch kein weiteres Bikarbonat in dem Behälter mehr auf. Setzt die Entnahme wieder ein, beginnt die Auflösung wieder nach Maßgabe der entsprechenden Menge an gesättigter Lösung. Die Auflösung des festen Bikarbonats geht also während der Dialyse fortlaufend in dem relativ kleineren Behälter unmittelbar am Dialysegerät vor sich.

Die Erfindung erstreckt sich auch auf einen Behälter, der an das geschilderte Verfahren in der in Anspruch 2 wiedergegebenen Weise angepaßt ist.

Der Behälter ist in der Betriebsstellung so angeordnet, daß der Filterboden horizontal steht, so daß das Trockenkonzentrat darauf liegen kann, und daß der Einlaß oberhalb und der Auslaß unterhalb des Filterbodens angeordnet sind. Das Wasser durchströmt das Trockenkonzentrat vertikal und löst dabei die Sättigungsmenge auf. Das Volumen des Behälters ist nicht besonders groß. Es ist durch die aufzunehmende Menge an Bikarbonat und eine Wassermenge bestimmt, die eine rasche gleichmäßige Bildung der gesättigten Bikarbonatlösung sowie eine störungsfreie Entnahme derselben aus dem Behälter gestattet. In Betracht kommen Behälter von etwa 0,5 bis 2 Liter Inhalt.

Der Filterboden kann gemäß Anspruch 3 durch einen keramischen Filter gebildet sein, dessen Porenweite gemäß Anspruch 4 100 bis 160 µm betragen kann.

Der Behälter kann am Einlaß mit einer abnehmbaren Anschlußtülle aus Glas versehen sein, die mit einem Überwurfgewindering festgezogen und mit einer elastischen Dichtung abgedichtet wird. Der Überwurfgewindering wird auf den Glasbehälter aufgeschraubt und verbindet die Anschlußtülle mit dem eigentlichen Glasbehälter. Ferner kann am unteren Teil des vorbekannten Glasbehälters der Flüssigkeitsauslaß durch eine mit dem Glasbehälter einstückige gläserne Anschlußtülle gebildet sein.

Die gläsernen Anschlußtüllen erfahren jedoch bei der Reinigung und sonstigen Handhabung, die meist in einem Becken stattfinden, leicht Beschädigungen durch Anstoßen. Gerade unbemerkte kleine Absplitterungen können gefährlich sein, wenn sie in den Kreislauf geraten. Sie hinterlassen außerdem rauhe Stellen, an denen sich Verunreinigungen und Keime festsezten können. Außerdem sind die Anschlußtüllen an Flüssigkeitseinlaß und Flüssigkeitsauslaß in der Herstellung aufwendig, wenn sie, ob einstückig oder separat, aus Glas gefertigt werden, da allenfalls Kleinserien in Betracht kommen.

Um einen Behälter zur Bereitstellung der Bikarbonatlösung für die Bikarbonat-Hämodialyse hinsichtlich des Einlasses und/oder des Auslasses einfacher und wirtschaftlicher auszugestalten und bei der Reinigung und sonstigen Handhabung Beschädigungen zu vermeiden, ist es gemäß Anspruch 5 vorteilhaft, eine einteilige Anschlußtülle aus Kunststoff für den Flüssigkeitseinlaß und/oder für den Flüssigkeitsauslaß zu wählen, die dem Nachteil der leichten Beschädigbarkeit bei der Reinigung nicht ausgesetzt ist und gegebenenfalls sogar ein im Handel erhältliches, an sich für andere Zwecke vorgesehenes Kaufteil sein kann.

Gemäß Anspruch 6 kann diese Anschlußtülle aufgeschraubt werden.

Die aus Kunststoff gefertigten Anschlußtüllen für Flüssigkeitseinlaß und für den Flüssigkeitsauslaß können gegenüber dem Glaskörper mit einem Dichtring abgedichtet werden (Anspruch 7) und als Verstärkung, zum Angriff bei der Handhabung und zum Schutz der Gewindestutzen des Behälters einen Umfangsrand aufweisen (Anspruch 8).

Es ist wichtig, daß in dem Behälter stets ein gewisser Füllstand aufrechterhalten und eine gewisse Mindestkontaktzeit des eingeleiteten Reinwassers mit dem festen Bikarbonat gewährleistet ist, damit sich die Lösung in dem Behälter tatsächlich sättigen kann.

Bei einem bekannten Dialysegerät, welches mit den bereits erwähnten Kartuschen arbeitet, ist ein geschlossenes Flüssigkeitssystem vorgesehen, bei welchem die entnommene Flüssigkeitsmenge aus dem Dialysegerät selbsttätig nachgeliefert wird. Dieses System ist aber an das spezielle Gerät angepaßt, und es lassen sich wegen der Halterungen nur ganz bestimmte Kartuschen verwenden.

Um den Gedanken der Bereitung einer gesättigten Bikarbonatlösung aus festem Bikarbonat am Dialysegerät für beliebige, mit einer außen bereitgestellten gesättigten Bikarbonatlösung arbeitende Dialysegeräte nutzbar machen zu können, ohne in die Technik dieser Geräte eingreifen zu müssen, kann eine Vorrichtung zur Durchführung des eingangs beschriebenen Verfahrens in der in Anspruch 9 wiedergegebenen Weise ausgestaltet sein.

Der Behälter und die Einrichtung zur Aufrechterhaltung eines bestimmten Flüssigkeitsniveaus sind beide außerhalb des Dialysegeräts angeordnet und bilden zusammen ein System, welches die ordnungsgemäße Bildung der gesättigten Lösung sicherstellt, weil immer Flüssigkeit über dem festen Bikarbonat steht und auf diese Weise eine gewisse Mindestkontaktzeit gegeben ist.

In einer ersten in Betracht kommenden Ausführungsform der Vorrichtung gemäß Anspruch 10 ist dem Behälter ein etwa in gleicher Höhe angeordneter Vorschaltbehälter mit einem Schwimmerventil zugeordnet, der mit dem Behälter nach Art kommunizierender Röhren zusammenwirkt, so daß in beiden stets das gleiche Flüssigkeitsniveau herrscht und die Schließstellung des Schwimmerventils in dem Vorschaltbehälter das Flüssigkeitsniveau in dem eigentlichen Behälter bestimmt.

Es ist aber auch möglich, gemäß Anspruch 11, die Einrichtung in den Behälter zu integrieren, indem in der Reinwasserzuleitung in dem Behälter ein Schwimmerventil angeordnet ist, welches eine Füllung bis zu einem bestimmten Niveau gestattet und verhindert, daß durch den Zulauf an Reinwasser fertige gesättigte Bikarbonatlösung am Flüssigkeitsauslaß herausgedrückt wird, ohne abgerufen worden zu sein.

Der Behälter kann für die Durchführung einer einzelnen Dialyse ausgelegt sein. Er kann aber auch gemäß Anspruch 12 größer bemessen sein, so daß in einem einzelnen Dialysegerät nacheinander mehrere Dialysen mit einer Füllung des Behälters durchgeführt werden können oder aber der Behälter an mehrere Dialysegeräte angeschlossen ist, so daß gleichzeitig mehrere Dialysen durchgeführt werden können.

In der Zeichnung sind Ausführungsbeispiele der Erfindung schematisch dargestellt.
Fig. 1 zeigt schematisch die Arbeitsweise bei der Herstellung von Dialysierflüssigkeit;
Fig. 2 zeigt eine Ansicht des Behälters zur Aufnahme des Trockenkonzentrats.
Fig. 3 und 4 zeigen Ansichten weiterer Ausführungsformen des Behälters;
Fig. 5 zeigt einen Längschnitt durch die auf den Einlaß und/oder Auslaß aufschraubbare Anschlußtülle;
Fig. 6 zeigt eine Ansicht einer ersten Ausführungsform der erfindungsgemäßen Vorrichtung mit Vorschaltbehälter;
Fig. 7 und 8 zeigen Ausführungsformen mit in den Behälter integriertem Schwimmerventil;
Fig. 9 zeigt eine Ansicht eines in Betracht kommenden Schwimmerventils.

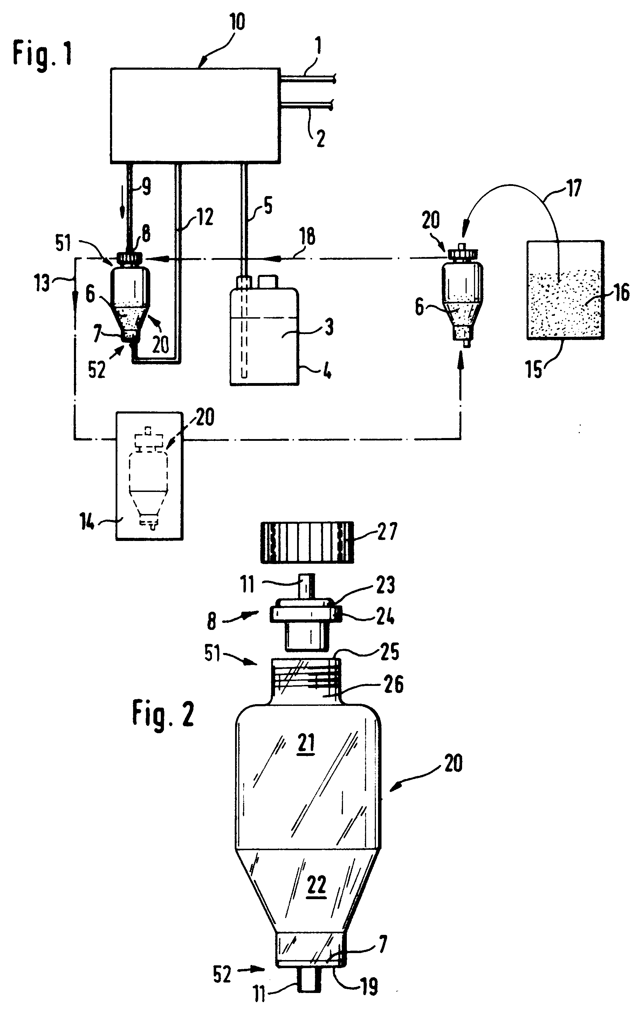

In Fig. 1 ist mit 10 das Dialysegerät bezeichnet, welches während der Dialyse am Bett des Patienten steht und über die Leitungen 1 und 2 mit dem Patienten verbunden ist. Über die Leitungen 1 und 2 wird das Blut des Patienten herangeführt und zurückgeleitet, welches in dem Dialysegerät der Dialysebehandlung, d.h. der Entfernung harnpflichtiger Bestandteile aus dem Blut unterworfen wird. Dies geschieht in dem nicht dargestellten sogenannten Dialysator, z.B. einem Kapillardialysator, bei welchem eine Membran vorhanden ist, an deren einer Seite das Blut und an deren anderer Seite die Dialysierflüssigkeit ansteht und durch die hindurch aus dem Blut die zu entfernenden Stoffe in die Dialysierflüssigkeit hinüberdiffundieren.

Bei der Bikarbonat-Hämodialyse wird die Dialysierflüssigkeit aus zwei Komponenten bereitet, nämlich aus einem das Bikarbonat enthaltenden Teilkonzentrat und einem Ionen wie Na⁺, K⁺, Ca²⁺ und Mg²⁺ enthaltenden weiteren Teilkonzentrat. Letzteres wird in Gestalt eines flüssigen Konzentrats 3 in Kanistern 4 von beispielsweise zehn Litern Inhalt angeliefert und von dem Dialysegerät 10 über eine Leitung 5 direkt aus dem Kanister 4 entnommen.

Das das Bikarbonat enthaltende Teilkonzentrat wird aus einer trockenen bzw. festen Bikarbonatzusammensetzung 6 hergestellt, welche sich in einem Behälter 20 aus Glas von etwa 1 Liter Inhalt auf einem horizontalen Filterboden 7 mit feinen Poren von 100 bis 160 µm Porenweite in einer für eine Dialyse berechneten Menge befindet. Oberhalb des Filterbodens 7 besitzt der Behälter 20 einen Anschluß 51, in dem dargestellten Fall einen Einlaß, unterhalb des Filterbodens 7, einen Anschluß 52 mit einem Anschlußstück 8, unterhalb des Filterbodens 7 einen Anschluß 52. Der Anschluß 51 umfaßt ein Anschlußstück 8 mit einem Schlauchstutzen 11, über den über eine durch einen Schlauch gebildete Leitung 9 aus dem Dialysegerät 10 durch Umkehrosmose gereinigtes Reinstwasser zugeleitet wird. Wenn von "oben" und "unten" die Rede ist, so ist stets die Stellung des Behälters 20 im Betrieb gemeint.

Das über die Leitung 9 eingespeiste Reinstwasser durchsetzt das Bikarbonat 6 und löst dabei soviel von dem Bikarbonat 6 auf, daß sich eine gesättigte Lösung ergibt, die bei Raumtemperatur etwa 8,4% Natriumbikarbonat enthält und über die Leitung 12 in das Dialysegerät 10 gelangt, wo die Vermischung mit dem über die Leitung 5 herangeführten anderen Flüssigkonzentrat und die Verdünnung auf die benötigte physiologische Konzentration erfolgen.

Der Behälter 20 ist wiederverwendbar. Er wird nach Abschluß einer Dialyse, wie es durch den Pfeil 13 angedeutet ist, in eine Reinigungs- und Sterilisiervorrichtung 14 überführt und anschließend aus einem größeren, z.B. in einem 25 kg-Sack 15 vorhandenen Vorrat 16 an das Natriumbikarbonat enthaltendem Trockenkonzentrat mit einer für die nächste Dialyse berechneten Menge an Bikarbonat 6 befüllt, was durch den Pfeil 17 angedeutet sein soll. Nach der Wiederbefüllung wird der Behälter 20 wieder im Sinne des Pfeiles 18 an das Dialysegerät 10 angeschlossen und erneut im bereits beschriebenen Sinne eingesetzt.

Die in Fig. 2 wiedergegebene Ausführungsform des Behälters 20 weist einen im wesentlichen zylindrischen Vorratsraum 21 für das Trockenkonzentrat auf, welcher sich nach unte in einem konischen Teil 22 auf etwa die Hälfte seines Durchmessers verjüngt. Am unteren Ende des konischen Teils 22 ist der als keramische Fritte ausgebildete und in den Behälter 20 am Rand dicht eingesetzte Filterboden 7 angebracht. Der Filterboden 7 überdeckt den gesamten Querschnitt des Vorratsraums 21, so daß das Wasser bzw. die Lösung den Weg durch die Poren des Filterbodens 7 hindurch nehmen muß und keine Nebenwege vorhanden sind. Dicht unterhalb des Filterbodens 7 ist am unteren Ende des Behälters 20 ein aus Glas bestehender Auslaßboden 19 mit einem Schlauchstutzen 11 für den Anschluß der durch einen Schlauch gebildeten Leitung 12 angeordnet.

Das aus Glas bestehende Anschlußstück 8 ist am oberen Ende des zylindrischen Teils 21 vorgesehen und umfaßt einen scheibenförmigen Umfangsvorsprung 23, der über eine elastische Dichtung 24 auf der Stirnseite 25 eines Gewindestutzens 26 des zylindrischen Teils 21 aufliegt und mittels eines Überwurfgewinderings aus Kunststoff 27 darauf dichtend festziehbar ist. Der Gewindestutzen 26 hat einen relativ großen Querschnitt, damit ein großer Einfüllquerschnitt für das Trockenkonzentrat 16 freigebbar ist. Im Betrieb ruht die abgemessene Menge des Bikarbonats 6 auf dem Filterboden 7 und wird von oben nach unten von dem an dem Anschluß 51 eingeleiteten Reinstwasser durchströmt. Die sich bildende gesättigte Bikarbonatlösung 57 tritt an dem Anschluß 52 aus.

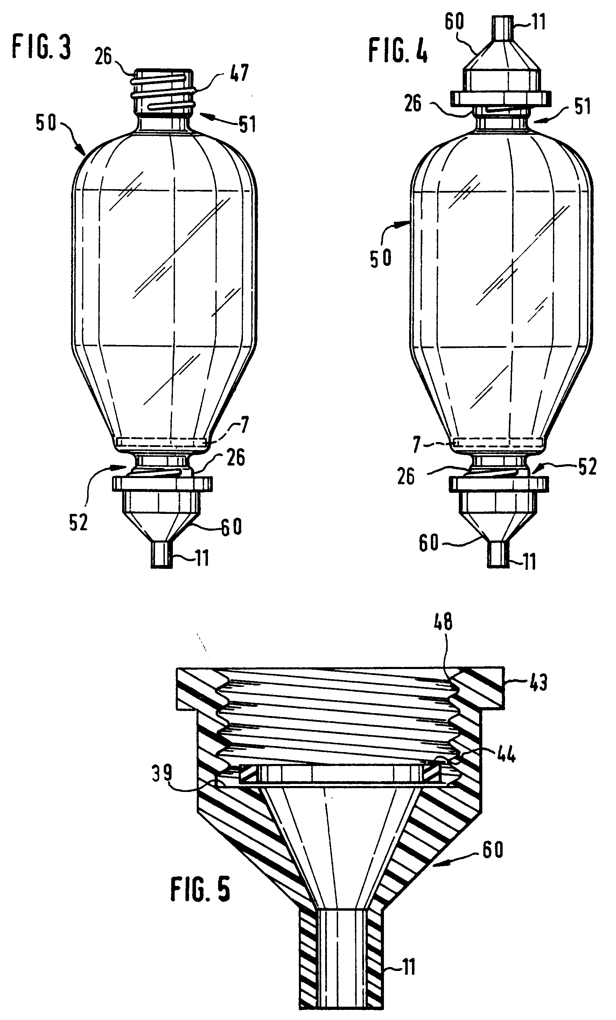

In den Fig. 3 bis 5 ist mit 50 ein weiterer Behälter zur Bereitstellung der bei der Bikarbonat-Hämodialyse benötigten Bikarbonatlösung am Dialysegerät bezeichnet. Im Ausführungsbeispiel nach Fig. 3 sind nur der Anschluß 52, Ausführungsbeispiel nach Fig. 2 sowohl der Anschluß 51 als auch der Anschluß 52 mit einer Anschlußtülle 60 aus einem geeigneten Kunststoff versehen. Als Kunststoff kommen dabei diverse Polymere in Frage; in dem Ausführungsbeispiel wurde Polyäthylen gewählt.

Der aus Glas mit einer Wandstärke von etwa 3 mm gefertigte und entsprechend stabile Behälterkörper ist an beiden Anschlüssen 51, 52 mit Gewindestutzen 26 versehen und so ausgebildet, daß die Anschlußtüllen 60 handelsübliche Gewindetüllen mit einem axialen Schlauchstuzen 11 für das Aufschieben eines nicht dargestellten Anschlußschlauches sein können. Zudem sind Flüssigkeitseinlaß und Flüssigkeitsauslaß gleich und die Anschlußtüllen 6O austauschbar. Die Anschlußtüllen 6O für Flüssigkeitseinlaß und Flüssigkeitsauslaß werden mit einem Dichtring 44 - im Ausführungsbeispiel aus Silikon - abgedichtet, der zwischen einer Innenschulter 39 des Anschlußstutzens 6O und der Stirnseite des jeweiligen Gewindestutzens 26 zusammengedrückt wird. An dem in Fig. 5 oberen, im Betrieb dem Behälter 5O zugewandten Rand weist die Anschlußtülle 6O einem radial nach außen vorspringenden Umfangsbund 43 zum Zwecke des Angriffs beim Drehen, der Verstärkung und des Schutzes des Gewindestutzens 26 auf. Anstelle der Verschraubung ist auch eine andere Technik des Aufsetzens des Anschlußstutzens 6O, z.B. Verklemmen oder Verschließen mit einem Bajonettverschluß möglich. Die Anschlußtüllen 6O sind im Ausführungsbeispiel hitzebeständig bis zur notwendigen Temperatur für die Sterilisation.

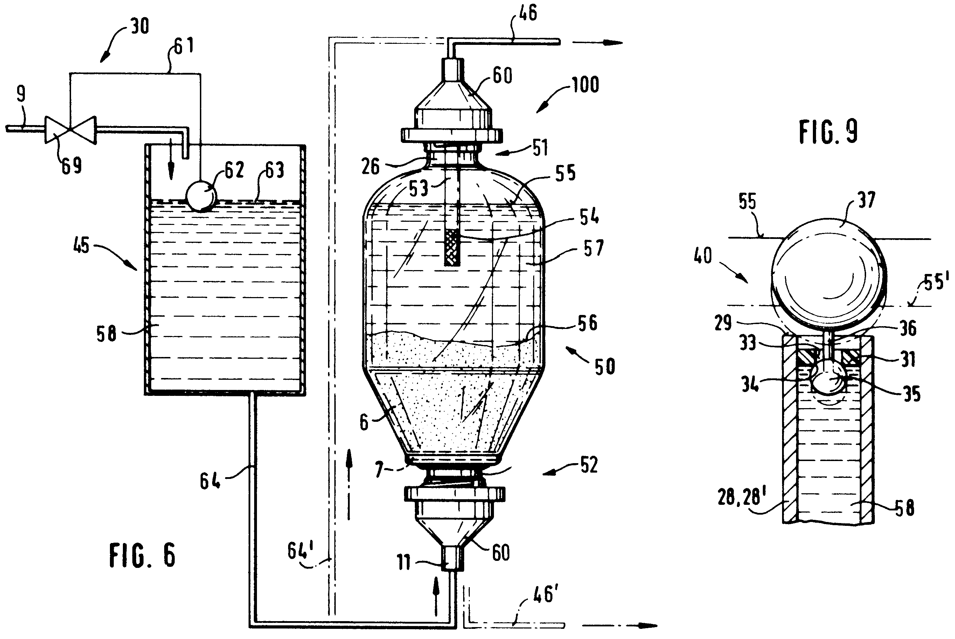

Die in Fig. 6 als Ganzes mit 1OO bezeichnete Vorrichtung umfaßt einen der vorbeschriebenen Behälter 5O aus Glas und einen Vorschaltbehälter 45 mit einem Schwimmerventil 3O. Der Behälter 5O und der Vorratsbehälter 45 sind zusammen etwa in gleicher Höhe außerhalb des hier nicht dargestellten Dialysegeräts angeordnet. Der Behälter 5O weist einen in dem Ausführungsbeispiel unten gelegenen Flüssigkeitseinlaß an dem Anschluß 52 und einen oben gelegenen Flüssigkeitsauslaß an dem Anschluß 51 auf, auf deren Anschlußstutzen 11 ein nicht dargestellter Anschlußschlauch aufschiebbar ist. Am oberen Ende geht der Mittelteil des Behälters 5O in einen gleichen Gewindestutzen 26 über, der den Flüssigkeitsauslaß bildet und auf den eine Anschlußtülle 60 aus Kunststoff aufgeschraubt ist, die ein durch das Innere des Gewindestutzens 26 in das Innere des Behälters 5O eingreifendes Tauchrohr 53 mit einem dessen Einlaßöffnung überdeckenden Filtereinsatz 54 trägt.

In dem unteren Teil des Behälters 5O liegt auf dem Filterboden 7 festes Bikarbonat 6 in Form eines Granulats bis zu einer oberen Begrenzung 56 auf.

Über die Leitung 9 wird durch Umkehrosmose gereinigtes Reinwasser 58 zugeleitet, welches in den Vorschaltbehälter 45 gelangt. Das Schwimmerventil 3O umfaßt ein Abschaltventil 69, welches über einen mit ihm mechanisch über ein Gestänge 61 verbundenen Schwimmer 61 betätigt wird. Bei einem bestimmten Flüssigkeitsniveau 63 wird das Abschaltventil 69 geschlossen, so daß das Flüssigkeitsniveau 63 nicht höher ansteigen kann und das mit dem Bikarbonat 6 in Berührung stehende Volumen auf einen definierten Wert begrenzt ist. Die Zuleitung des Reinwassers über die Leitung 9 erfolgt unter der Wirkung einer Pumpe oder unter Schwerewirkung aus einem höher angeordneten Vorratsbehälter.

Das Reinwasser 58 in dem Vorschaltbehälter 45 tritt durch die Leitung 64 aus dessen unterem Bereich in den Flüssigkeitseinlaß an dem Anschluß 52 über, durchsetzt den Filterboden 7 und die darüber lagernde Menge an Bikarbonat 6 und steigt sodann bis zu einem Flüssigkeitsniveau 55 an, welches mit dem Flüssigkeitsniveau 63 übereinstimmt, wie es bei kommunizierenden Röhren der Fall ist. Wird über das Tauchrohr 53 und die Leitung 46 die gebildete über dem festen Bikarbonat stehende gesättigte Bikarbonatlösung 57 entnommen, sinken das Flüssigkeitsniveau 55 und damit das Flüssigkeitsniveau 63 ab, und es wird durch Öffnung des Abschaltventils 69 Reinwasser nachgeliefert, bis das durch die Lage des Schwimmers 62 bestimmte ursprüngliche Flüssigkeitsniveau 63 wieder erreicht ist. Auf diese Weise steht an der Leitung 46 stets gesättigte Bikarbonatlösung 57 zur Verfügung, ohne daß darauf geachtet werden muß, daß der Behälter 1O nicht leerläuft oder gesättigte Bikarbonatlösung 57 von selbst durch die Leitung 46 herausgedrückt wird.

In dem gezeigten Ausführungsbeispiel ist der Flüssigkeitseinlaß unten und der Flüssigkeitsauslaß oben vorgesehen. Beide können jedoch auch vertauscht werden, wie durch die strichpunktiert angedeutete Führung der Leitung 64' und der Leitung 46' wiedergegeben sein soll.

Die für eine (oder mehrere) Dialyse(n) berechnete Menge an festem Bikarbonat 6 wird zu Beginn durch den Anschluß 51 eingefüllt. Am Schluß der Dialyse ist das feste Bikarbonat gerade verbraucht, so daß keine Verluste eintreten. Der Behälter 5o kann nach erfolgter Reinigung und Sterilisation erneut verwendet werden.

Die ganze Vorrichtung 1OO ist als separate Einheit außerhalb des Dialysegeräts angeordnet und kann bei jeder Art von Dialysegerät verwendet werden, ohne daß es irgendwelcher Probleme mit der Medizingeräte-Verordnung bereitender Eingriffe in die Dialysegeräte bedarf.

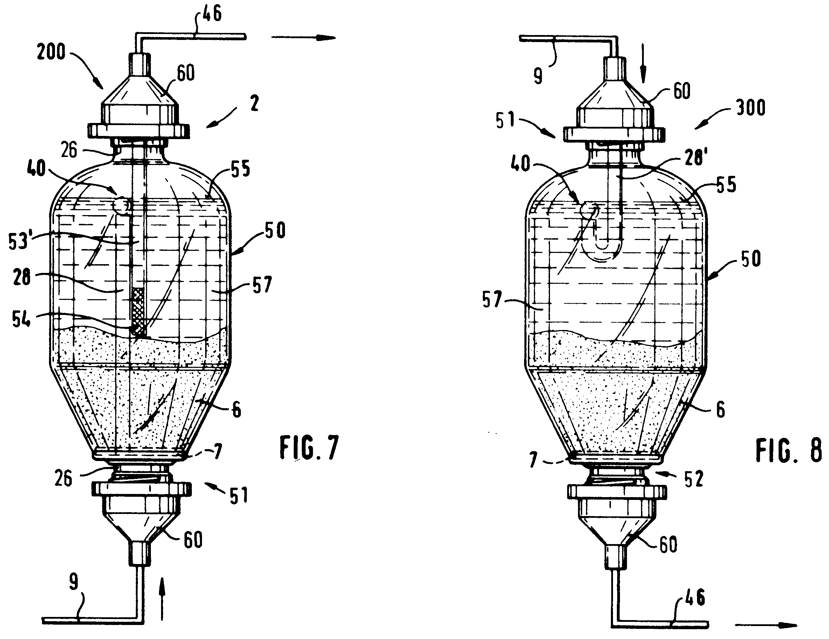

In den Fig. 7 und 8 sind Vorrichtungen 2OO bis 3OO dargestellt, bei denen das das Flüssigkeitsniveau 55 steuernde Schwimmerventil in den Behälter 5O integriert ist. Es sind hierbei also keine externen Vorschaltbehälter oder dergleichen erforderlich. Der Behälter 5O ist gleich ausgebildet wie in Fig. 6. Soweit im übrigen funktionell gleiche Teile vorkommen, sind die Bezugszahlen gleich.

Bei der Vorrichtung 2OO der Fig. 7 sind der Flüssigkeitseinlaß wieder unten und der Flüssigkeitsauslaß oben gelegen. Die untere Anschlußtülle 6O ist jedoch gegenüber der Anschlußtülle 6O der Fig. 6 insofern modifiziert, als sie ein Steigrohr 28 trägt, welches durch den unteren Gewindestutzen 26 hindurchführt und bis in die Höhe des angestrebten Flüssigkeitsniveaus 55 reicht. Am oberen Ende trägt das Steigrohr 28 ein Schwimmerventil 4O, welches in Fig. 9 im einzelnen dargestellt ist. Das durch die Leitung 9 herangeführte Reinwasser kann dank des Schwimmerventils 4O nicht höher steigen, als es dem Flüssigkeitsniveau 55 entspricht.

Auch die auf den oberen Gewindestutzen 26 aufgeschraubte Anschlußtülle 6O ist gegenüber der Anschlußtülle 6O der Fig. 6 modifiziert, indem das Tauchrohr 53' mit dem Filtereinlaß 54 tiefer in den Behälter 5O eintaucht, um einen gewissen Abstand der Entnahmestelle zu der Flüssigkeitszuführung an dem Schwimmerventil 4O zu schaffen.

Bei der Vorrichtung 3OO der Fig. 8 sind Flüssigkeitseinlaß und Flüssigkeitsauslaß in ihrer Lage vertauscht. Die Zuführleitung 9 für Reinwasser gelangt also von oben über die Anschlußtülle 6O in das Innere des Behälters 5O. Die Anschlußtülle 6O trägt ein U-förmig gebogenes Rohr 28', welches am Ende des aufwärts gebogenen U-Schenkels das Schwimmerventil 4O trägt, welches das Flüssigkeitsniveau 55 festlegt. Die gebildete gesättigte Bikarbonatlösung wird am unten gelegenen Flüssigkeitsauslaß über die Anschlußtülle 6O und die Leitung 46 abgezogen. Ein besonderer dem Filtereinlaß 54 entsprechender Filter kann hier entfallen, weil diese Aufgabe durch den Filterboden 7 übernommen wird.

In Fig. 9 ist eine mögliche Ausführungsform des Schwimmerventils 4O schematisch angedeutet. Das aufwärtsgerichtete Ende der Rohre 28 bzw. 28' ist quer zu ihrer Achse mit einer horizontalen Endfläche 29 versehen. In das Ende ist ein Dichtring 31 eingesetzt, zum Beispiel eingeklemmt oder eingeklebt, der eine zentrale Öffnung 33 und am unteren Rand der Öffnung eine Dichtfläche 34 aufweist. Die Dichtfläche 34 wirkt mit einer Dichtkugel 35 zusammen, die einen größeren Durchmesser als die Durchgangsöffnung 33 aufweist. Über einen Verbindungszapfen 36, der einen deutlich kleineren Durchmesser als die Durchgangsöffnung 33 besitzt, ist die Dichtkugel 35 mit einem in dem Ausführungsbeispiel kugelförmigen hohlen Schwimmer 37 verbunden, dessen Durchmesser größer als der Innendurchmesser der Rohre 28,28' ist und der außerhalb der Endfläche 29 derselben angeordnet ist.

Wenn das Flüssigkeitsniveau in dem Behälter 1O die erwünschte Lage 25 erreicht hat, zieht der Schwimmer 37 die Dichtkugel 35 hoch und sperrt die Durchgangsöffnung 33. Das in den Rohren 28,28' anstehende Reinwasser 58 kann nicht mehr nachströmen.

Sinkt das Flüssigkeitsniveau beispielsweise bis in die Höhe 25', so sinkt der Schwimmer 37 mit, wodurch die Dichtkugel 35 von der Dichtfläche 34 abhebt und den zwischen dem Verbindungszapfen 36 und dem Innenumfang der Durchgangsöffnung 33 verbleibenden Ringraum als Durchtrittsquerschnitt für das Reinwasser 58 freigibt. Der Schwimmer kann dabei bis zum Aufsitzen auf der Endfläche 29 absinken. Wegen seines geringen Gewichts wird das Nachströmen des Reinwassers nicht behindert. Liegt aber die Dichtkugel 35 wieder an der Dichtfläche 34 an, ist die Dichtwirkung nicht mehr von der Tragkraft des Schwimmers 37 abhängig, sondern ist die Anordnung selbstdichtend, d.h. auch bei einer Druckerhöhung des Reinwassers 58 bleibt die Dichtigkeit vorhanden. Der abgesunkene Zustand des Schwimmers 37 und der Dichtkugel 35 ist in Fig. 9 gestrichelt angedeutet.

## Patentansprüche

1. Verfahren zur Bereitstellung der bei der Bikarbonat-Hämodialyse benötigten Bikarbonatlösung am Dialysegerät (1O), bei welchem eine in einem Behälter (2O,5O) vorhandene für eine Dialyse (oder mehrere Dialysen) berechnete Menge einer festen Bikarbonatzusammensetzung (6) mit gereinigtem Wasser zu einer konzentrierten Lösung aufgelöst wird, worauf der Behälter (2O,5O nach Verbrauch der Menge gereinigt und sterilisiert und dann aus einem größeren am Dialyseort vorhandenen Vorrat der festen Bikarbonatzusammensetzung (6) mit einer vorausberechneten Menge derselben wiederbefüllt wird,
dadurch gekennzeichnet,
daß die feste Bikarbonatzusammensetzung in dem am Dialysegerät befindlichen und mit diesem unmittelbar verbundenen Behälter (2O,5O) ständig in Kontakt mit einem begrenzten Volumen des gereinigten Wassers gehalten und in diesem bis zur Sättigung der gebildeten Lösung aufgelöst wird
und daß die gesättigte Bikarbonatlösung (57) von dem Dialysegerät (1O) bedarfsweise aus dem Behälter (2O,5O) entnommen wird, wobei während der Dialyse ständig eine Wiederauffüllung durch gereinigtes Wasser bis zu dem begrenzten Volumen und eine fortschreitende Auflösung der Bikarbonatzusammensetzung (6) bis zur Sättigung der Lösung erfolgen.

2. Behälter zur Verwendung bei dem Verfahren nach Anspruch 1, dadurch gekennzeichnet, daß er durch einen Behälter (2O,5O) aus Glas mit einem ersten Anschluß (51) an einem Ende und einem zweiten Anschluß (52) am anderen Ende und mit einem sich dazwischen in dem Behälter (2O,5O) quer zur Strömung der Flüssigkeit erstreckenden, den Querschnitt des Behälters (2O,5O) ganz überdeckenden Filterboden (7) gebildet ist.

3. Behälter nach Anspruch 2, dadurch gekennzeichnet, daß der Filterboden (7) durch einen keramischen Filter gebildet ist.

4. Behälter nach Anspruch 3, dadurch gekennzeichnet, daß die Porenweite des Filterbodens (7) 1OO bis 16O µm beträgt.

5. Behälter nach einem der Ansprüche 1 bis 4, dadurch gekennzeichnet, daß an dem den Flüssigkeitseinlaß und/oder an dem den Flüssigkeitauslaß bildenden Anschluß (51,52) eine aus einem Kunststoffteil gebildete Anschlußtülle (6O) angebracht ist.

6. Behälter nach Anspruch 5,
dadurch gekennzeichnet,
daß die Anschlußtülle (6O) mit einem Innengewinde (48) versehen ist,
daß der Flüssigkeitseinlaß und/oder der Flüssigkeitsauslaß des Behälters (5O) durch einen Gewindestutzen (26) mit einem dem Innengewinde (48) entsprechenden Außengewinde (47) gebildet sind
und daß die Anschlußtülle (6O) auf den jeweiligen Gewindestutzen (26) aufgeschraubt ist.

7. Behälter nach Anspruch 5 oder 6, dadurch gekennzeichnet, daß die Anschlußtülle (6O) mit einer Dichtung (44) gegenüber dem Gewindestutzen (26) abgedichtet ist.

8. Behälter nach einem der Ansprüche 5 bis 7, dadurch gekennzeichnet, daß die Anschlußtülle (6O) an ihrem bei aufgeschraubter Anschlußtülle (6O) dem Behälter (5O) zugewandten Rand einen nach außen vorspringenden Umfangsbund (43) aufweist.

9. Vorrichtung zur Durchführung des Verfahrens nach Anspruch 1 mit einem Behälter nach einem der Ansprüche 2 bis 8, dadurch gekennzeichnet, daß sie eine Einrichtung (9) zur Zufuhr von Reinwasser (58) zum Flüssigkeitseinlaß und eine Einrichtung (46) zur Entnahme gesättigter Bikarbonatlösung (57) am Flüssigkeitsauslaß aufweist und dem Behälter (5O) eine außerhalb des Dialysegeräts vorgesehende Einrichtung zur Aufrechterhaltung eines bestimmten Flüssigkeitsniveaus (55) in dem Behälter (5O) zugeordnet ist.

10. Vorrichtung nach Anspruch 9, dadurch gekennzeichnet, dßa die Einrichtung durch einen in der Reinwasserzuleitung (9) auf etwa gleicher Höhe mit dem Behälter (5O) außerhalb desselben angeordneten Vorschaltbehälter (45) mit einem Schwimmerventil (3O) gebildet ist, welcher mit dem Flüssigkeitseinlaß des Behälters (5O) derart verbunden ist, daß der Behälter (5O) und der Vorschaltbehälter (45) kommunizierende Röhren bilden.

11. Vorrichtung nach Anspruch 9, dadurch gekennzeichnet, daß die Einrichtung durch ein in der Reinwasserzuleitung (9,28;9,28') in dem Behälter (5O) angeordnetes Schwimmerventil (4O) gebildet ist.

12. Vorrichtung nach einem der Ansprüche 9 bis 11, dadurch gekennzeichnet, daß der Behälter (5O) für eine Menge an festem Bikarbonat (6) ausgelegt ist, die für mehrere an einem Dialysegerät hintereinander oder mehrere an verschiedenen Dialysegeräten gleichzeitig durchgeführte Dialysen ausreicht.
